Europäisches Patentamt

European Patent Office

Office européen des brevets

(11) Publication number: **0 643 307 A1**

# EUROPEAN PATENT APPLICATION

(21) Application number: **93500124.8**

(22) Date of filing: **14.09.93**

(51) Int. Cl.6: **G01N 33/58**, G01N 33/532

(43) Date of publication of application:
**15.03.95 Bulletin 95/11**

(84) Designated Contracting States:
**AT BE CH DE DK ES FR GB GR IE IT LI LU MC NL PT SE**

(71) Applicant: **CENTRO DE INGENIERIA GENETICA Y BIOTECNOLOGIA**
**Avenida 31,**
**Cubanacan Playa**
**Havana (CU)**

(72) Inventor: **Santiago Laecaille, Carlos A.**
**Calle 184, n 3112**
**Apto. 14,**
**Playa,**
**Ciudad Habana (CU)**
Inventor: **Lescaille Buitrago, Lissett de la C.**
**Calle 184, n 3112**
**Apto. 14,**
**Playa,**
**Ciudad Habana (CU)**

(74) Representative: **Gil-Vega, Victor**
**Estébanez Calderon, 3 - 5B**
**E-28020 Madrid (ES)**

(54) **Visual immunoassay method for the detection of ligands, based in the use of opaque plastic supports.**

(57) The present invention is related to the field of immunology, and particularly with an immunoassay method ligand detection in fluid bodies, using plastic supports.

The technical objective of this invention consists of a manual immunoassay method which provides high sensitivity, and specificity, and reagent economy, for the detection of ligands in fluid bodies, using opaque white plastic supports. The latter contain either antigens or antibodies, immobilized in low - depth microwells. The detection of the respective ligand binding partner (antibodies or antigens, respectively) is evidenced using reagents conjugated with monodisperse colloidal gold, being the signal amplified posteriorly "in situ" by physical developers based on silver ions. The samples, and all liquid reagents are placed in contact with the microwells, finally obtaining metallic coloured insoluble reactions of very high contrast, that can be easily interpreted in a visual fashion.

This method can be employed for the detection of any type of antigen (including small molecules with discrete epitope structure) or antibody.

Rank Xerox (UK) Business Services
(3. 10/3.0 9/3.3.4)

Infectious, acute, and chronic diseases of humans, animals, and plants can be diagnosed or detected using immunologically-based assays. A variety of such procedures are known, and include the so-called "sandwich" immunoassays, the competitive binding assays, and others, that can be employed to determine the presence or absence of a molecule of interest in fluid or non fluid samples. A common feature of immunoassays is that they are based on the specificity of antigen-antibody reactions. This specific reaction gives rise to what is denominated an immunocomplex. The antibodies (also known as immunoglobulins) can be of polyclonal or monoclonal origin, and act as specific binding partners for particular antigens, being the latter molecules with single or multiple epitopic sites. Not only non-immunoglobulin molecules, but also a second antibody, can be an antigen in a given situation. Immunoassays are also characterized by the presence of some kind of labelling procedure that allows the identification of an antigen-antibody reaction. The labelling can be part of this antigen-antibody reaction, or constitute an additional step in the assay. In the latter case, labelling involves an additional specific recognition of either the antigen or antibody in the immunocomplex by an additional molecule bearing a marker substance. A wide variety of markers are currently employed in immunoassays (radioactive isotopes, metal and non-metal particles that can agglutinate, substances that can be activated for light emission, or that can give rise to colored soluble or insoluble reactions. etc.).

Horisberger and Rosset, (J. Histochem. Cytochem. 25:295-305 1977), describe an agglutination assay using colloidal gold as a label. In spite of the fact that the assay is simple and economic, it has disadvantages associated with the extended assay time, and an important subjective component in determining the final point of the reaction through color appreciation, among other aspects.

Leuvering (U.S. Patent No. 4,313,734), describes a new immunoassay procedure involving the use of colloidal metal substances. While such colloids are employed as labels in immunological conjugates, the procedure is dependent upon the use of spectrophotometers for the identification of the presence of the colloidal metal, after it has been chemically extracted from the site of the immunological reaction. The assay thus relies on cumbersome preliminary extraction procedures, and expensive auxiliary instrumentation, in order to obtain relatively insensitive results.

European Patent Application EP 158,746, assigned to Janssen, describes an immunoassay procedure based on the diffusion of reagents on membrane matrix surfaces (immunobloting techniques). While providing a better alternative over that of Leuvering, the described method still suffers from excessive execution time and stages, including the previous preparation and blocking of the membranes.

In the European Patent Application EP 29,397, Janssen takes into consideration existing knowledge about the use of physical developers based on the reduction of silver ions, for the visualization of insoluble metals in tissues (Danscher, Histochemistry 71:1-16, 1981), and the use of colloidal gold particle conjugates in histological staining, that are afterwards amplified with similar developers (Holgate et all. J. Histochem. Cytochem. 31,938-944, 1983), and provide a way to obtain higher sensitivity than the original method of immunostaining with colloidal gold conjugates. In the said patent, Janssen describes the additional use of physical developers, insensitive to light and conformed by two separate solutions, in his procedure, as a way to improve sensitivity and visual characteristics of the assays based on membrane matrixes.

Chan et al, reported in the Abstracts of the 1986 Meeting of the American Society of Microbiologists (C-213 on page 363, and C-409 on page 396) the use of colloidal gold as a marker in assays, using a format very similar to that discovered by Janssen. The Chun et al. assays, however, required significant incubation times, on the order of 2-3 hours, in order to achieve acceptable levels of sensitivity.

The European Patent Applications EP 250,137 and EP 258,963, assigned to Ortho Diagnostic Systems Inc., describe a new immunoassay procedure that uses colloidal gold as marker reagent, and a special device that contains a membrane matrix with an immobilized immunologically active component. While the main claims of this procedure are centered on the sensitivity and assay speed achieved when the fluid samples and reagents are made to traverse the membrane, the method has several disadvantages. The membrane matrixes require previous steps of preparation and blocking, and the use of a special individual device for each sample, composed of the said membrane, absorbent material, and a conformed plastic body, leads an increase in cost and technological complexity in production. Unexpected difficulties can arise during the visual reading of the results, due to interference of unspecific background, that can affect the quality, and increase the subjective component in the interpretation of the reaction by the end user, specially in the case of low reactive samples, and/or when using specific biological fluids as total blood, serum, plasma, etc. Finally, and of special relevance, the individual format of such method does not allow the development of true negative and positive sample controls in the same device where the unknown sample is tested.

The present invention relates to the immunoassay methods and specially, with a new immunoassay system that detects immunologically active molecules in fluid samples. Such molecules can be single or monoepitopic antigens, or antibodies. As the specific recognition between antigens and antibodies creates an indistinctive ligand-ligand binding partner pair, the latter terms will be hereafter used indistinctively in the text. These terms will also be used to describe the reaction between a labelled substance and the immunocomplexes.

In particular the present invention has as object a new assay procedure, that uses opaque white plastic supports with delimited reaction areas, coated with a ligand binding partner. The immunological reaction takes place when the sample is incubated in such areas, and the specific binding partner coated to the plastic support reacts with the ligand present in the sample. The labelling of such reaction is made possible using conjugates of monodisperse colloidal gold particles and a specific binding partner for the ligand in the sample. The colloidal gold particles have a preferred diameter range between 1 and 18 nanometers, and the conjugate an absorbance value between 0.5 and 1.15, measured at a wave length of 520-540 nanometers. The visualization of the reaction is made after a final step where the signal produced by the deposition of the colloidal gold is amplified by physical developers, based on silver ions. This amplification gives rise to a metallic darkish-black color, that contrasts with the white plastic support.

The new immunoassay system involves:
- a plastic opaque white support with polished delimited reaction surfaces, coated with a ligand binding partner. The ligand binding partner will be interpreted hereafter as either of molecular homogeneity or as a heterogenous mixture of molecules;
- contacting the said delimited reaction surfaces with a sample that contains a ligand specific for the binding partner coated onto the plastic surface. The ligand will be interpreted hereafter as either of molecular homogeneity or as a heterogenous mixture of molecules;
- adding either simultaneously or successively a direct or indirect conjugate of colloidal gold and a binding partner, specific for the ligand contained in the sample;
- contacting the delimited reaction surfaces with a mixture of liquid reagents that give rise to a physical developer containing silver ions, able to amplify signal produced by the colloidal gold particles;
- observing the said delimited reaction surfaces for the presence of a metallic darkish-black colored precipitate, indicative of the presence or absence of the desired ligand in the sample.

Overall, this immunoassay procedure is one of easy preparation, execution, reagent economy, high sensitivity and specificity levels, and that can be adequated to formats that assure the inclusion of true negative and positive sample controls in each experiment. The procedure can be adapted to "sandwich", competitive binding, or other types of assays, for the detection of either antigens or antibodies in fluid sample. Such immunoassays can be produced economically, with the necessary quality requirements of batch production. Another advantage of the procedure proposed in this invention is that it gives rise to immunoassays that can be fully interpreted in a visual manner, without the need of additional equipment.

Another aspect of the present invention is the possible extension of the nature of the support to several plastic substances of varying polymeric structure as polystyrene, polyamide, polycarbonate, polyvinyl chloride, and others, that mixed in the stage of raw material, and in adequate proportion, with chemical compounds of titanium, zinc and others, result in opaque supports that can be casted in different final formats. With further application of surface treatments, including those similar to the ones conventionally employed in the production of microtiter well plates and strips for enzyme linked immunosorbent assays (ELISA) techniques, it is possible to optimize the coating of the plastic surfaces for a variety of ligands, either by adsorption, or covalent linkage.

In correspondence with the characteristics of the present invention, the new immunoassay procedure is useful for the detection of a wide variety of ligands in fluid samples, such as total blood, serum, plasma, urine, saliva, and others. Specific antigens of, or antibodies to, viral entities (human immunodeficiency viruses, hepatitis C and B viruses, cytomegalovirus, rotavirus, etc.), bacteria and parasites (Neisseria meningitidis, Leptospirae, Toxoplasma gondii, Treponema pallidum, etc.) can be detected. Ligands of different nature to the ones described above, i.e. other biological and non-biological molecules, can also be detected using immunoassays constructed on the principles of the present invention.

As briefly mentioned above, the assay uses opaque white polystyrene supports, with delimited polished circular reaction areas, where a ligand binding partner is coated. The details of the procedure depend on the type of assay, whether this be a direct, competitive, or "sandwich"-type of assay. In general, the immunological reaction is produced when the support is incubated with the sample, that contains a ligand specific for the binding partner coated to the plastic surface. A successive incubation with a conjugate of colloidal gold and binding partner for the ligand (sandwich-type) follows. In the competitive-binding type of assay, this step is unnecessary, as the sample is incubated with the coated plastic surface, together with a conjugate of colloidal gold and the ligand of interest. A final step in the method involves the incubation of the plastic surfaces with a mixture of liquid reagents that give rise to a physical developer containing silver ions, able to amplify the final detection of the colloidal gold particles. Washing steps after each incubations ensure the elimination of the excess of ligands and unspecific molecules present in the sample, of unbound conjugate, and physical developers solution.

Surprisingly, it was observed that the produced silver metallic particles remain nucleated surrounding the gold particles coupled to the polystyrene surface, due to the presence of the ligand-ligand binding partner-conjugate reaction. The result is a metallic darkish-black color on the reaction arcas, that can easily be visualized due to the contrast with the white plastic support. In "sandwich"-types of assays, the intensity of the color depends on the amount of ligand present in the sample. In competitive binding assays, the amount of ligand in the sample will be proportional to a decrease in color. In the cases where no specific immunological reaction occurs in the surface, metallic reduction of the silver and nucleation will not occur, and the surfaces remains uncolored.

As a result of the present invention, significantly smaller amounts of ligand binding partner is needed for the coating of the plastic reaction surface, in comparison with methods based on membrane matrixes, or conventional ELISA. Also, the volumes of other liquid reagents can be reduced. The immunoassay procedure also needs a lower amount of sample per test than that commonly employed in ELISA and in many membrane-based techniques.

The present invention has shown that the combination of polished plastic surfaces of opaque white color, and the use of colloidal gold conjugates, followed by physical developers, can result in very simplified, yet highly sensitive immunoassay procedures, of acceptable operational speed (around 60 minutes of total assay time), and allowing a visual reading of results.

Such results had not been previously possible. For example, in the European Patent Applications EP 250,137 and 258,963 assigned to Ortho Diagnostic Systems Inc., it is documented that assays on plastic transparent surfaces, using exclusively colloidal gold conjugates, were insufficient for the visual detection of the immunological reactions.

Also it was surprisingly found that a combination of plastic supports of opaque white color, obtained with a mixture of the polymeric material and substances like titanium dioxide, conjugates based on colloidal gold particles of small diameters (1-18 nanometers), and physical developers, will allow a totally adequate "in situ" visualization of many immunological reactions between ligands and ligand binding partners. The rose colored signals produced in the reaction surface by the colloidal gold, are amplified by way of an additional short incubation with the physical developers based on silver ions. The signal amplification is due to the formation of a highly contrasting metallic darkish-black color that develops when the gold particles, specifically linked to the plastic surface by the immunological reaction, induce the reduction of the silver ions to the corresponding metal, that deposits in a proportional and controlled fashion around the gold particles. This amplification makes possible the visualization of the reaction in cases where the ligand concentration in the sample is very low. Thus, the sensitivity and specificity levels obtained through the said procedure are comparable with those reported for conventional immunoenzymatic techniques (ELISA).

Generically, immunoassays intended for the detection of antibodies (now the ligand) against known pathogens (serologic diagnosis) in fluid sample, are some of procedures that can be developed using the principles described in this invention. For example, using a mixture of recombinant proteins representative of the envelope gp 120 and gp41 antigens, and the core gag24 protein of the human immunodeficiency virus (HIV-1), produced by the Center for Genetic Engineering and Biotechnology of Havana, Cuba, we have developed a "sandwich" format that allows the serological detection of IgG antibodies to HIV-1 in samples from seropositive subjects. The preparation of the said recombinant proteins is a known art and has not to be described here. It is possible to coat minimal amounts of the said recombinant proteins on the delimited circular reaction polished areas of a polystyrene opaque white supports that have the shape of a slide. The said plastic support has been prepared as mentioned before to obtain the opalescence and white color, and can be homogeneously activated using gamma irradiation, in order to increase the efficiency of coating of a variety of molecules of different size and structure. The processes of gamma irradiation of polymeric plastic structures for activation is also known art and does not require a detailed description.

These types of new serologic immunoassays have also been prepared for other infectious agents i.e. IgG, IgA, and IgM to Toxoplasma gondii, IgG and IgM to Rubella, IgG and IgM to Cytomegalovirus, IgG and IgM to Leptospira, IgG to Hepatitis C virus, IgG to Hepatitis B virus, IgG and IgM to Treponema pallidum, etc.

In all said cases, direct or indirect conjugate of colloidal gold particles and a specific binding partner for the said immunoglobulins would be used for the labelling of the immunological reaction. Such binding partners can be monoclonal or polyclonal antibodies specific to IgG, IgA or IgM, protein A, protein G, or any other immunoglobulin-specific binding partner. In the particular example of the application of this invention to the serological detection of IgG antibodies to HIV-1, the employed conjugate was constructed by coupling the colloidal gold particles and recombinant protein A. Recombinant protein A is produced by the Center for Genetic Engineering and Biotechnology of Havana, Cuba, and its preparation is a known art. Consequently it was considered unnecessary a detailed description. This reagent is an example of a ligand able to specifically recognize and bind to the Fc region of several immunoglobulins, among them, the human IgG (Romano et al. Immunochemistry 14:711, 1977).

Independently of the mentioned examples of serologic immunoassays, it is possible to prepare other "sandwich"-type of assays, where monoclonal and polyclonal antibodies, insted of antigens, are coated to the said plastic reaction surface. In this way, a variety of specific polyepitopic antigens (now the ligands) can be detected in fluid samples (i.e. Hepatitis B virus surface antigen, Cytomegalovirus, Rotavirus, Herpes simplex I and II, Chlamydia, blood lipoproteins, etc.). In such cases the colloidal gold particles are conjugated with other monoclonal and polyclonal antibodies that identify either similar or different epitopes of the antigen that has been "captured" by the ligand binding partner coated to the plastic surface. The final step of the assay, i.e. the amplification of the signal with physical developers, is similar to what has been described above.

Another advantage of this invention over previous methods is that the amount of sample that has to be employed for the assay is very small. The size of the circular delimited reaction areas in the plastic support can be made of a diameter such that only 20-30 microliters of diluted blood, plasma, serum, urine, etc., has to be applied. The inventors have found that even with delimited areas of as little as 7 mm of diameter the reaction can be readily visualized, with the required sensitivity levels, in "sandwich"-types of assays.

As the present invention employs colloidal gold, it was surprisingly found that using conjugates prepared with colloidal gold particles of diameter ranging between 1 and 18 nanometers, diluted to an optimal absorbance value of 1 at a wave length of 520-540 nm, low sample volumes as those referred (20-30 microliters), and short incubation times at room temperature for sample (5-30 minutes), conjugate (5-30 minutes), and physical developers (5-15 minutes) were enough to achieve high sensitivity levels.

Ideally, the best physical developers are the light insensitive ones of the type described by the European Patent Application EP 29,397 assigned to Janssen. In the example mentioned above, related to the serologic detection of IgG antibodies to HIV-1, the assay is finalized by applying over the reaction areas an equal part mixture of a reagent A, containing silver ions stabilized in a transparent solution, and a reagent B, containing the developer in itself, also in a transparent solution. In the absence of gold metallic particles in the plastic surface, the mixture of reagents A and B will conserve its transparency for a prolonged time, under environmental light. No color will thus develop in the reaction areas.

The procedure described in the present invention can also be adapted to cases where "sandwich"-types of format cannot be developed, due to the absence of multiple epitopic sites in the substance of interest (i.e. drugs, hormones, chemical compounds, etc.). For such situations, competitive assays can be developed; the plastic support is coated with a specific ligand binding partner (i.e. monoclonal or polyclonal antibodies, etc.), and the sample presumably containing the ligand is placed in contact with the reaction areas, simultaneously with a conjugate of such ligand and colloidal gold.

The procedure object of the present invention can give rise to an immunoassay that can be packed as a diagnostic/detection kit containing the precoated opaque white plastic supports, a vial with the colloidal gold conjugate, the vials with the solutions for the preparation of the physical developer, flasks with the necessary dilution and wash buffers, vials with true positive and negative controls, instruction sheet, and ancillary materials (spare vials, disposable pipettes, etc.). The format of the plastic support can be adapted to various requirements, as, for example, for multiple tests (slide-like supports with several reaction areas, or any other convenient geometry), or for a single-test assay.

Additionally, the present invention can be applied in combination with adequate instrumentation to develop a quantitative assay, based on densitometric reading, or using sensors specific for the involved metallic substances.

## EXAMPLES

The following examples are to be understood as ways to illustrate the invention, and should not be interpreted in any way as limiting its applicative projection and scope.

## Example 1: Preparation of the colloidal gold solution

All the glass surfaces that contact the colloidal gold solution, or the components for its preparation, should be silanized using a 2% dimethylchlorosilane solution (Merck) in chloroform (Fluka), and a 20 minute immersion, followed by successive washes in absolute ethanol and distilled water. A 1% gold chloride

EP 0 643 307 A1

(Aldrich) solution, prepared in a final volume of 1 liter of Milli-Q (Millipore Inc.) water, is heated to 100$\underline{o}$C for 10 minutes. Forty ml of a mixture of trisodium citrate and 1% tannic acid, as reducing agent, are added to the reactor. After a boiling period of approximately 5-10 minutes, the formation of the colloidal gold solution occurs, indicated by the successive changes in color. The reactor is then slowly cooled at room temperature. The average diameter of the colloidal gold particles produced under such conditions varies between 10 and 15 nanometers, and the monodisperse character and homogeneity were determined using transmission electron microscopy (100 kV, 50,000x) employing hydrophilic mesh with Formvar-carbon membranes.

The pH of the colloidal solution is adjusted with the addition of 0.1 M K2CO3. For conjugation with recombinant protein A (produced by the Center for Genetic Engineering and Biotechnology of Havana, Cuba), the pH value is adjusted in the 6.0-6.5 range. When coupling polyclonal antibodies, the pH range should be 7.0-7.5. In the case of monoclonal antibodies, antigens, or any other type of ligand, the isoelectric point of each particular molecule should be taken into account. In general, the optical density of the colloidal suspension is measured at a wave length of 520-540 nanometers.

**Example 2: Coupling of ligands to colloidal gold**

Procedure 1.- Adsorptive coupling of recombinant Protein A

Salt-free recombinant protein A was diluted with a 0.005 M solution of NaCl, to a concentration of 1 mg/ml. The "gold number", that is to say, the amount of protein A necessary to stabilize 100 ml of colloid, was determined by serial dilution of the protein in fixed volumes of colloidal suspension. The optimal concentration of recombinant protein A was calculated as the minimal necessary concentration, plus a 10% excess, to stabilize 1 liter of colloidal gold. To each liter of stabilized colloidal gold, PEG 20,000 (Fluka) was added to a final 1%, and then centrifuged at 4$\underline{o}$C in a 5% glycerol (Fluka) gradient, for 45 minutes, at 35,000 x G. The supernatant was discarded and the pellet washed twice in the 5% glycerol gradient, suspended in a 1:10 ratio with respect to the starting colloidal gold concentration, and filtered through a 0.45 m membrane (Sartorius). The final conjugate concentration was adjusted at a OD of 1 (wave length of 540 nanometers) using 0.1 M phosphate buffer, pH 7.4, containing 1% RIA grade bovine serum albumin (BSA; BDH), and 0.05 M sodium azide (Merck). The suspension was stored at 2-8$\underline{o}$C.

Similar procedures of adsorptive coupling were developed for other ligands with known isoelectric points, such as monoclonal antibodies, viral antigens, etc.

Procedure 2.- Covalent coupling of polyclonal antibodies

To a 1 liter volume of colloidal gold solution, generated following the conditions mentioned in Example 1, and at pH 6.0, 1 g of RIA grade BSA, prediluted in 10 ml of filtered distilled water, was added. After two hours of stirring at room temperature, the solution was filtered using a 0.2 m membrane (Sartorius), and centrifuged at 4$\underline{o}$C for 45 minutes, and 35,000 x G. The supernatant was discarded and the pellet washed with distilled water using similar centrifugation conditions. The O.D. was adjusted to 1 (wave length of 520-540 nanometers). To each 100 ml of the BSA-colloidal gold conjugate, glutaraldehyde was added to a final 1% (w/v), and mixed for two hours at room temperature. The activated particles were incubated overnight at 2-5$\underline{o}$C with a buffer phosphate solution, pH 7.4, containing 1 mg of the purified polyclonal antibodies, and the covalent links stabilized with 1 mg of sodium borohydrate (Aldrich). After an incubation period of 30 minutes with constant stirring, 5 ml of buffer-BSA-glycine solution, pH 8.0, was added, and the mixture centrifuged at 5,000 x G for 45 minutes, followed by two washings with 0.1 M phosphate buffered saline (PBS), pH 8.0. The final precipitate is suspended in PBS containing 0.05 M triethanolamine, 0.2% BSA, and 0.1% sodium azide, adjusted at a O.D. of 5 (wave length of 540 nanometers), and stored between 2-4$\underline{o}$C.

**Example 3: Preparation of the physical developer**

Procedure 1.- Preparation of the physical developer, sensitive to environmental light

Following similar principles to those described by Danscher (Histochem. Cytochem. 31, 938-944, 1983).
Solution A is prepared in a volume of 100 ml of deionized water, by adding the following components:
- 2.55 g of trisodium citrate
- 2.35 g of citric acid

- 0.85 g of hydroquinone

Solution B is prepared in a volume of 100ml of distilled and deionized water, just before use, and carefully protected from light, by adding 0.11 g of silver lactate.

The physical developer is prepared by mixing the total of both solutions A and B, in conditions protected from environmental light.

All opaque plastic white slides used in the testing (see examples), that contained colloidal gold particles coupled to the surface due to the existence of a ligand-ligand binding partner immunological reaction, were immersed during 10 minutes in the physical developer. In the presence of small metallic particles, the silver ions of the developing solution were capable of reducing to the corresponding metal, and form metallic precipitates of darkish-black color. This amplification effect of the signal increases considerably the contrast, and thus, the detection sensitivity of the method.

Procedure 2.- Preparation of a system of physical developer, insensitive to environmental light

Following similar principles as those described, by Janssen (EP 29,397).

Solution A is prepared in a 100 ml volume of distilled and deionized water, with the following components:
- imidazole (BDH) 6 g
- sodium formiate (BDH) 0.85 g
- silver nitrate (Merck) 0.18 g

The final pH of the solution should be 8.0.

Solution B is prepared in a 100 ml volume of distilled and deionized water, with the following components:
- imidazole (BDH) 2 g
- hydroquinone (BDH) 1.5 g
- sodium sulfite (BDH) 1 g
- formic acid (BDH) 2.5 ml

The final pH of the solution should be 5.0.

Both solutions are separately stored between 2 and 4ₒC and maintain their transparency and stability to environmental light for periods of time up to 12 months.

The preparation of the physical developer is made just before its use. Equal amounts of solutions A and B are mixed at room light and temperature conditions, for a final volume enough to cover the circular delimited reaction areas in the plastic supports of the aforementioned examples, and incubated for 10 minutes at room temperature. In the presence of the small gold particles, the silver ions of the developing solution are capable of reducing to the corresponding metal, and nucleate surrounding the gold particles, to form metallic precipitates of darkish-black color. This amplification effect of the signal increases considerably the contrast, without unspecific background effects, and thus, confers to the method a high level of sensitivity.

**Example 4: Preparation of slide-like plastic supports**

Procedure 1.- Opaque white polystyrene supports

Approximately 25 kg of granular polystyrene (BASF 158K, low density, crystal grade) is mixed and homogenized with 10-100 g of titanium dioxide and 1-20 g of zinc stearate. Through a casting procedure, disposable plastic supports, of opaque white color are obtained, with similar format to that of microscopy slides, and containing multiple (8-12) circular delimited (7-9 mm of diameter) low-depth wells (reaction areas) of polished surface (see enclosed annex with drawings of this and other formats). The said supports are activated by gamma irradiation, using total doses of 0.1-1.0 megaRAD, and stored at room temperature, under 50-70% relative humidity, and with the reaction areas well protected from damage.

Procedure 2.- Opaque-white polyamide supports

Approximately 25 kg of granular polyamide (NYLON-66) is mixed and homogenized mechanically with 10-100 g of titanium dioxide. Through a casting procedure, disposable plastic supports, of opaque white color are obtained, with a format of strips attached to vial screw caps (see enclosed annex with drawings of this and other formats). The said supports are activated by gamma irradiation, using total doses of 0.1-1.0 megaRAD, and stored at room temperature, under 50-70% relative humidity, and with the reaction areas well protected from damage.

**Example 5: Assay for the detection of antibodies against the human immunodeficiency virus type 1 (HIV-1)**

Procedure 1.- Preparation of the plastic supports containing the immobilized ligand binding partner

Using supports as those described in Procedure 1 of Example 4 (slide-like support with 12 circular delimited reaction areas), the low-depth wells are sensitized with a mixture of recombinant proteins representative of antigenic domains of the envelope gp120 and gp41 proteins, and the core gag24 proteins of HIV-1 (the recombinant proteins are produced in E. Coli in the Center for Genetic Engineering and Biotechnology of Havana, Cuba). The recombinant proteins are diluted in a carbonate-bicarbonate buffer, pH 8.0. Each well is coated with 50 l of the mixture in concentrations ranging from 1 to 20 g/ml, for 3 hours, at 37oC.

The supports are washed with PBS-0.05% Tween 20 and 0.1% of sodium azide to remove the excess of non adsorbed protein, dried, and stored between 2-4oC, protected from humidity with desiccant.

Procedure 2.- Sample preparation

Five hundred forty five samples were studied, distributed as follows:
- 150 total blood samples from HIV-1 seropositive individuals, previously characterized by enzyme immunoassay methods (UMELISA, TecnoSUMA, Havana, Cuba, and ELISA RECVIH, Heber Biotec S.A., Havana, Cuba), and confirmatory tests (Western blot, Dupont de Nemours, DAVIH).
- 200 serum samples from blood donors, of which 198 had been previously classified as negative, and 2 as positives for antibodies against HIV-1, by the aforementioned screening immunoenzymatic techniques. The 2 positive samples were afterwards confirmed as false positives by Western blot.
- 195 serum samples from patients arriving to a clinical laboratory of a hospital. Of these, 193 were classified as negative, and 2 as positive, by the aforementioned screening immmunoenzymatic techniques. The 2 positive samples were afterwards confirmed as false positives by Western blot.

Each sample was diluted 1:10 with a dilution buffer containing 0.1 M PBS, 0.05% M Tween 20, 0.1% sodium azide, and 0.5% diethanolamine.

Procedure 3.- Execution of the assay

1) Each diluted sample was applied in 20 l volumes to the reaction areas of the 12-well polystyrene plastic slides, prepared as described in Procedure 1 of Example 4, and incubated at room temperature for 20 minutes.

2) The slides were washed briefly with distilled water, then with PBS-Tween 20 during 1 minute, and finally drained of excess liquid using filter paper.

3) Each reaction area was covered with 20 l of a conjugate of recombinant protein A and monodisperse colloidal gold (15 nm), prepared as detailed in Procedure 1 of Example 2, and incubated for 20 minutes at room temperature.

4) The excess of reagents was eliminated by washing, as described in 2).

5) The following step of the assay was a 10-minute incubation of the reaction areas with 20 l of the physical developer, prepared just before use, and following the method described in Procedure 2 of Example 3.

6) The plastic slides were briefly washed with distilled water, dried, and the reading of results made by simple visual inspection. In the case of positive reactions, the circular areas turned to a metallic darkish-black color, with an intensity of the signal varying in accordance with the concentration of specific anti-HIV-1 antibodies, present in the samples. In negative cases, the reaction areas remain white.

Procedure 4.- Sensitivity and specificity studies

Screening of total blood and serum samples for IgG antibodies to HIV-1.

| Origin | n | seropos. | seroneg. | sensitiv. | specif. |
|---|---|---|---|---|---|
| Sanatorio S.V.* | 130 | 150 | 0 | 100% | - |
| Blood Bank** | 200 | 2(FP) | 198 | - | 99% |
| C.García Hosp.*** | 195 | 2(FP) | 193 | - | 98% |

* Samples of seropositive individuals (groups II and III) and patients with AIDS or AIDS-related complex (group IV), Havana, Cuba.
** Samples from seronegative donors from the Marianao Blood Bank, Havana, Cuba.
*** Unclassified samples evaluated in the clinical laboratory of the Cira García Hospital, Havana, Cuba.
(FP): Samples detected as false positives by the immunoassay procedure object of this invention, as well as through the conventional screening techniques (UMELISA and RECVIH). Western blots confirmed that these samples were negative.

**Example 6: Assay for the simultaneous detection of IgG, IgM, and IgA antibodies to Toxoplasma gondii**

Procedure 1.- Preparation of the plastic supports containing the immobilized ligand binding partner

The supports were used according to Procedure 1 of Example 4 (3-well strip format; see enclosed annex with drawings). The supports were sensitized in similar conditions to the ones described in Procedure 1 of Example 5, but using as antigen a lysate containing membrane and cytoplasmic proteins of the trophozoite of Toxoplasma gondii, obtained from the ascitic fluid of BALB/c mice, inoculated with the RH strain.

Procedure 2.- Sample preparation

For demonstrative purposes, we studied human serum samples previously characterized through reference serology techniques (indirect immunofluorescence, IIF) for the presence of IgG, IgM, and IgA antibodies, specific to Toxoplasma gondii. The samples were previously diluted with PBS-Tween 20 in the same conditions required for IIF techniques:
- Initial dilution of 1:32 for IgG antibodies.
- Initial dilution of 1:8 for IgM antibodies.
- Initial dilution of 1:4 for IgA antibodies.

In the latter two cases, the samples were preadsorbed with appropriate amounts of a desiccated Protein A-sepharose gel, so as to remove competitive effects of specific IgG antibodies.

Procedure 3.- Execution of the assay

The assay was developed following basically the steps described in Procedure 3 of Example 5, exception made of the facts that:
(a) one same diluted sample, as described in Procedure 2, was serially diluted three times, and incubated in separate wells of a plastic support with three delimited circular areas (see annex).
(b) different conjugates were used to demonstrate the presence of each specific immunoglobulin type:
- Colloidal gold (15 nm)-anti human IgG (sheep).
- Colloidal gold (15 nm)-anti human IgM (sheep).
- Colloidal gold (15 nm)-anti human IgA (sheep).

The sheep antibodies, purified by affinity chromatography, were obtained in the Center for Genetic Engineering and Biotechnology of Havana, Cuba, and the coupling method was the one described in Example 2 for covalent binding.

<u>Procedure 4.-</u> <u>Analysis of results</u>

The results were considered positive for a given antibody type, when the assayed sample, conveniently diluted, gave rise to a metallic darkish-black color in the delimited reaction areas.

In the cases where the delimited reaction areas remained white, or staining was evidently lower than comparative strips incubated with diluted positive controls for each immunoglobulin type, the results were considered negative.

1. Determination of immunoglobulin titers (G, M, and A) in serum samples:

| Clinical reference* | n | IgG | IgM | IgA | Interpretation |
|---|---|---|---|---|---|
| Pregnancy | 3 | 1:32 | - | 1:16 | Infection relapse |
|  |  | 1:32 | 1:8 | - | Subacute primary infection |
|  |  | 1:256 | 1:32 | 1:16 | Acute primary infection |
| Abortion | 3 | - | 1:8 | 1:8 | Acute primary infection |
|  |  | 1:32 | 1:16 | - | Subacute primary infection |
|  |  | 1:256 | 1:16 | 1:16 | Acute primary infection |
| Eye disorders | 5 | 1:32 | 1:8 | 1:8 | Acute primary infection |
|  |  | 1:32 | 1:8 | 1:8 | Acute primary infection |
|  |  | 1:128 | - | 1:16 | Relapse or reinfection |
|  |  | 1:128 | 1:8 | 1:64 | Acute primary infection |
|  |  | 1:256 | 1:16 | - | Subacute primary infection |

\* Interpretation criteria according to Van Knapen and Panggabean (J. Clin. Microbiol. 6:545-547, 1977).

2. Results of the secreening of sera from individuals classified according to clinical picture:

| Clinical group | n | positive (%) | negative (%) |
|---|---|---|---|
| Eye disorders | 5 | 4 (80%) | 1 (20%) |
| General disorders | 8 | 8 (100%) | - - |
| Ginecol./obst. disorders | 42 | 28 (66.6%) | 14 (33.4%) |
| No reference | 79 | 50 (63.3%) | 29 (36.7%) |
| Total | 134 | 90 (67.1%) | 44 (32.9%) |

3. Sensitivity and specificity values, with respect to the conventional reference IIF techniques:

| Sensitivity (S) | 94.2 % |
|---|---|
| Specificity (SP) | 100 % |
| Positive predictive value (PPV) | 100 % |

Calculations were developed following a 2x2 table:

|          | Immunoassay |     | | A: | positivos coincidentes |
|----------|:-----------:|:---:|-|----|------------------------|
|          | +           | −   | | B: | False negatives |
|          |             |     | | C: | False positives |
| IFI    + | A           | B   | | D: | Coincident negatives |
|          |             |     | | A + B: | Positives by IIF |
|        − | C           | D   | | C + D: | Negatives by IIF |
|          |             |     | | A + C: | Positives by the immunoassay |
|          |             |     | | B + D: | Negatives by the immunoassay |

$$S = A \times 100 / A + B$$
$$SP = D \times 100 / C + D$$
$$PPV = A \times 100 / A + C$$

**Claims**

1. Immunoassay method for the detection of ligands characterized in that it comprises:

   a.- An opaque white plastic support delimited and polished surfaces, coated with a ligand or pairs of ligands.

   b.- A sample that contains a ligand or pairs of ligands, this sample contacting with said plastic surface and simultaneously or successively with a conjugate of colloidal gold and a ligand or pairs of ligands, in which a immunological reaction occurs.

   c.- A reactive containing a ligand or pairs of binding ligands, conjugated, directly or indirectly, with colloidal gold.

   d.- Liquid reactive agents consisting in a physical developer that contains silver ions able to improve the final detection of the colloidal metallic particles when contacting with said plastic surface.

2. Immunoassay method according to claim 1 characterized in that the ligand is an antigen.

3. Immunoassay method according to claim 1 characterized in that one of the ligands of the ligant binding partners of step a) is a specific immunoglobuline for the antigen of claim 2.; wherein one of the ligands of the ligand binding partners of step b) is a ligand, conjugated directly or indirectly with colloidal gold, which competes with the ligand of the sample and which is applied simultaneously with the sample.

4. Immunoassay method according to claim 1, characterized in that the ligand is an antigen which contains a single epitopic site.

5. Immunoassay method according to claim 1 characterized in one of the ligands of the ligand binding partners of step a) the antigen of claim 4 which has the same immonological reactivity as the antigen contained in the sample; and in that one of the ligands of the ligand binding partners in step b) is an antibody conjugated directly or indirectly with colloidal gold, which is specific for the antigen contained in the sample; and in that the reactive and the sample are mixed prior to their simultaneously application to the plastic surface.

6. Immunoassay method according to claim 1, characterized in that the ligand is an antigen which has at least two epitopic sites.

7. Immunoassay method according to claim 1 characterized in one of the ligands of the ligand binding partners of step a) is a specific immunoglobine for the first epitopic site of the antigen of claim 6; and of the ligands of the ligand binding partners of step b) is a specific immonoglobuline for the second epitopic site of the antigen; the second immunoglobuline being directly or indirectly conjugated to colloidal gold.

8. Immunoassay method according to claim 1, characterized in that one of the components of the ligand binding partners of step a) is an antigen that has the same immunological reactivity than the antigen contained in the sample; and in that one of the ligands of the ligand binding partnes of step b) is an antibody conjugated directly or indirectly with colloidal gold, which is specific for the antigens.

9. Immunoassay method according to claim 1, characterized in that the ligand is an antibody.

10. Immunoassay method according to claim 1, characterized in that one of the pairs of ligands of step a) is an antigen for which the antibody is specific; and that one of the ligands of the ligand binding partners of step b) is a immunoglobuline, or protein A, or proteine G conjugated directly or indirectly with colloidal gold, whic is specific for the antibody.

11. Immunoassay method according to claim 1, characterized in that the utilized colloidal gold suspension has an optical density between 0.5 and 1.5, measured at 540 nm.

12. Immunoassay method according to claim 1, characterized in that the colloidal gold particle has a diameter in the range of 1-18 nm.

13. Immunoassay method according to claim 1, characterized in that the white opaque plastic support is prepared based on a mixture of different polymeric substances such as polystyrene, polyvynil chloride, polycarbonate, polyamide, and others, conventionally used to obtain conformed rigid surfaces.

14. A kit to be used in the immunoassay method of claim 1; characterized in that it comprises: a white opaque plastic support coated with a ligand binding partner for the reaction with the ligand to be detected; a container that contains a reactive which comprises the conjugated colloidal gold and the ligand to react with the other ligand corresponding to the binding ligand pair on the plastic surface in a competitive assay, or for a ligand contained in the fluid sample in the "sandwich" type assay; a container for each of the liquid reactives which previously mixed conform the physical developer based on silver ions for the final amplification of the visually detected signal.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO-A-91 01003 (SECRETARY OF STATE FOR HEALTH IN HER BRITANIC MAJESTY'S GOVERNMENT OF) <br> * the whole document * <br> --- | 1-14 | G01N33/58 <br> G01N33/532 |
| D,X | EP-A-0 158 746 (JANSSEN PHARMACEUTICA N.V.) <br> * page 4, line 7 - page 5, line 7 * <br> * page 7, line 6 - line 23 * <br> * page 10, line 18 - page 11, line 16 * <br> --- | 1-14 | |
| X | EP-A-0 293 947 (JANSSEN PHARMACEUTICA N.V.) <br> * column 17, line 35 - column 20, line 58 * <br> --- | 1-14 | |
| X | EP-A-0 401 913 (JANSSEN PHARMACEUTICA N.V.) <br> * the whole document * <br> --- | 1-14 | |
| A | WO-A-92 11537 (NYCOMED PHARMA AS ET AL.) <br> * the whole document * <br> --- | 1,11,12 | TECHNICAL FIELDS SEARCHED (Int.Cl.6) |
| A | DATABASE WPI <br> Derwent Publications Ltd., London, GB; <br> AN 81-92053D <br> & JP-A-56 140 346 (MITSUBISHI PAPER MILL) <br> 2 November 1981 <br> * abstract * <br> --- | 1,13 | G01N |
| A | EP-A-0 106 324 (MINNESOTA MINING AND MANUFACTURING COMPANY) <br> * page 7, line 13 - page 9, line 9 * <br> ----- | 1,14 | |

The present search report has been drawn up for all claims

| Place of search | Date of completion of the search | Examiner |
|---|---|---|
| BERLIN | 12 April 1994 | De Kok, A |

EPO FORM 1503 03.82 (P04C01)